# NEW EUROPEAN PATENT SPECIFICATION

(11) **EP 1 320 356 B2**
(45) Date of publication and mention of the opposition decision: **08.06.2011**
(45) Mention of the grant of the patent: 12.12.2007
(21) Application number: 01967545.3
(22) Date of filing: 20.09.2001
(51) Int. Cl.: A61K 9/107, A61K 9/00, A23L 1/302, A23L 1/22, A61K 31/5513, A61P 3/02, A61K 31/55, A61K 31/10, A61K 31/58

(54) **PREPARATION OF VITAMIN EMULSIONS AND CONCENTRATES THEREOF**
HERSTELLUNG VON VITAMINEMULSIONEN UND KONZENTRATE DAVON
PREPARATION D'EMULSIONS ET DE CONCENTRES DE CES DERNIERES

(30) Priority: 20.09.2000 GB 0023067; 20.09.2000 GB 0023068; 20.09.2000 GB 0023069
(43) Date of publication of application: 25.06.2003
(73) Proprietor: Nycomed Pharma AS, 1385 Asker (NO)
(72) Inventor: SCHLYTER, Jimmy Hirschsprung, Dr ammensveien 852, N-1372 Asker (DK); PIENE, Jan Yngvar, Drammensveien 852, N-1372 Asker (NO)
(74) Representative: Plougmann & Vingtoft A/S
(86) International application number: PCT/GB2001/004231
(87) International publication number: WO 2002/024165

(56) References cited:
- EP-A- 0 278 103
- EP-A- 0 972 513
- EP-A- 0 987 008
- EP-A- 1 077 059
- WO-A-00/54838
- WO-A-92/14443
- WO-A-93/04598
- WO-A-97/35558
- DE-A- 19 756 123
- GB-A- 919 193
- US- - 5 232 733
- US-A- 4 497 800
- US-A- 4 801 455
- US-A- 5 192 577
- US-A- 5 393 461
- US-A- 5 508 041
- DATABASE BIOSIS [Online] BIOSCIENCES INFORMATION SERVICE, PHILADELPHIA, PA, US; 1993 JIZOMOTO HIROAKI ET AL: "Gelatin-acacia microcapsules for trapping micro oil droplets containing lipophilic drugs and ready disintegration in the gastrointestinal tract." Database accession no. PREV199396115711 XP008005312 & PHARMACEUTICAL RESEARCH (NEW YORK), vol. 10, no. 8, 1993, pages 1115-1122, ISSN: 0724-8741
- WIKIPEDIA CONTRIBUTORS: "Carrageenan" [Online] 8 March 2006 (2006-03-08), WIKIPEDIA, THE FREE ENCYCLOPEDIA , - Retrieved from the Internet: URL:http://en.wikipedia.org/w/index.php?ti tle=Carrageenan&oldid=42849289> [retrieved on 2006-03-10] * page 1, line 15 *
- BUDAVARI S. ET AL: 'The Merck Index. Eleventh edition', 1989, MERCK & CO., INC, RATHWAY, NJ, USA "Algin" * page 41, paragraph 231 *
- BUDAVARI S. ET AL: 'The Merck Index. Eleventh Edition', 1989, MERCK & CO., INC., RATHWAY, NJ, USA "Agar" * page 31, paragraph 172 *

## Description

This invention relates to a process for the manufacture of emulsions, for example, drug-containing formulations in liquid forms, for example a syrup of a liquid concentrate.

Many compositions, e.g. pharmaceuticals, cosmetics, nutraceuticals, etc. need to be formulated as emulsions, generally due to a necessary component being substantially water-insoluble or to the preference of the consumer for a liquid rather than a solid dosage form.

However formulating products in emulsion form raises its own problems, for example the stability of the emulsion itself and of its components.

In the case for example of vitamin-containing compositions (e.g. food supplements or nutraceuticals), formulation as a liquid composition with extended storage or shelf-life however provides its own problems, in particular relating to the stability of the vitamins. Vitamins in liquid dosage forms are easily degraded mainly due to the influence of temperature, moisture, oxygen, light and pH. The presence of other vitamins will also influence the degradation pattern of each individual vitamin which complicates the formulation task even further. It is also important to realize that it is the stability of the most unstable component which determines the overall shelf-life of a product.

US 5,192,577 relates to stable nutritional supplements in the form of oil-in-water emulsions. It is disclosed that addition of kappa-carrageenan in combination with Xanthan gum is essential in order to obtain a stabilizing effect of the emulsions. It is disclosed that the compositions of the invention remain stable after sterilization for at least 27 weeks at 22°C to 25°C.

GB 919,193 discloses a free flowing powdered product based on an emulsion comprising fat soluble vitamin and sucrose monoester of a fatty acid. It is disclosed that the sucrose monoester of a fatty has an excellent emulsifying power.

US 4,497,800 discloses a liquid diet composition wherein carrageenan is added to the oil phase as an emulsion stabilizer.

The problem posed by the instability of vitamins in liquid formulation is highlighted in "Oil and Water-Soluble Vitamins: Oral Solution" in the monograph for Nutritional Supplements in USP 24 which states that supplements should contain not less than 90% and not more than 250%, 150% or 450% of the labelled amounts of certain vitamins. Thus vitamin compositions generally contain more than the labelled amounts of certain vitamins to allow for degradation during storage and so meet the legal requirement that the vitamin content must be at least as high as indicated on the product label throughout the shelf-life of the product. While vitamin degradation may thus be compensated for, to some extent, by use of an overage, the overage should desirably be relatively small otherwise doses received when using relatively fresh product would be well above the desired level. The USP monograph thus sets out limits for the vitamin content in a liquid formulation which are undesirably wide from a legal and cost perspective. Much narrower limits are clearly desirable. There is thus a general need for a manner of producing emulsions or emulsion concentrates which have enhanced stability, and where appropriate enhance the stability of any degradable components for which the emulsion is a vehicle.

We have now found that oil-in-water emulsion compositions with very long storage lives can be produced using a combination of an emulsifier, agar agar as a gelling agent and a thickener as well as water and a water-immiscible liquid.

We have also found that such stable emulsions may successfully be concentrated (e.g. by drying) and subsequently reconstituted.

Thus viewed from one aspect the invention provides a process for the preparation of a liquid emulsion composition having a continuous aqueous phase containing a gelling agent and a thickener and optionally a physiologically tolerable amount of at least one water soluble vitamin and a discontinuous oil phase, comprising at least one lipophilic vitamin and optionally an edible triglyceride, said emulsion composition further containing at least one emulsifying agent, said process comprising:
forming an aqueous composition comprising an aqueous solution of a gelling agent and a thickener and optionally at least one water soluble vitamin;
forming a water-immiscible liquid composition comprising at least one emulsifying agent and at least one lipophilic vitamin;
mixing said water-immiscible composition with at least part of said aqueous composition whereby to form an oil-in-water emulsion; and
if required mixing further components with said emulsion whereby to form said liquid emulsion composition, and wherein the gelling agent is agar agar, and optionally, e.g. mixing in further aqueous or non-aqueous compositions containing a physiologically tolerable mineral (e.g. iron, zinc) compound, sweeteners, a further gelling agent or thickener, further vitamins, non-vitamin drugs, minerals, flavours, colours, preservatives etc.

At least one vitamin and optionally a non-vitamin drug should be present.

Viewed from a further aspect the invention provides a process for the manufacture of a liquid emulsion composition having a continuous aqueous phase containing agar agar as gelling agent, and one or more gum, for example, a plant gum, and optionally a physiologically tolerable amount of at least one water soluble vitamin and/or non-vitamin drug, and a discontinuous oil phase preferably comprising at least one lipophilic vitamin and optionally a non-vitamin drug and optionally an edible triglyceride, said emulsion composition further containing at least one emulsifying agent, preferably one selected from edible phospholipids and fatty acid esters, said oil phase comprising at least one of (i) droplets of a discontinuous aqueous phase containing a physiologically active or beneficial compound dissolved therein, (ii) an inorganic particulate, and (iii) a non-vitamin lipophilic drug compound.

In the case of a multivitamin and mineral-containing emulsion, the major proportion (i.e. at least 50% wt) of the oil phase in the compositions of the invention preferably comprises an edible oil (e.g. an edible triglyceride) and/or vitamin E. Vitamin E is especially preferred. Further lipophilic vitamins may be present in the oil phase. In the case of a drug-containing emulsion the vitamins may be replaced by a non-vitamin lipophilic drug compound.

In the compositions of the invention, when present, the edible triglyceride is preferably a fish oil or more preferably a plant oil, optionally wholly or partially hydrogenated, e.g. coconut oil, soyabean oil, rape seed oil, sunflower oil, safflower oil, mustard seed oil, olive oil, peanut oil, etc. Particularly preferably the oil will be one rich in relatively short fatty acid chains, e.g. having a high abundance of C₆ to C₁₈ or more preferably C₈ to C₁₂ fatty acid residues. Particularly preferably, the weight average fatty acid carbon content is in the range C₈ to C₁₂. Alternatively, the oil will be one rich in long fatty acid chains, e.g. having a high abundance of C₁₆ to C₂₂ or particularly C₁₈ to C₂₂ fatty acid residues, especially C₁₈. Fatty acid profiles can be adjusted as desired by fractionating plant oil or by mixing plant oils from different sources. Highly unsaturated fatty acids are in general not preferred.

The oil phase, e.g. the edible triglyceride when present and vitamin E or a non-vitamin lipophilic drug substance, together preferably constitute up to 20% by weight of the total composition, for example, up to 10%, more preferably up to 5% by weight of the total composition, more especially preferably up to 3% by weight, still more preferably up to 1% by weight, e.g. 0.05 to 0.5% by weight.

The vitamin E used according to the invention may be in any of the forms in which vitamin E may be presented including derivatives, analogues, metabolites and bioprecursors, e.g. α-tocopherol, α-tocopherol acetate, α-tocopherol acid succinate, vitamin E TPGS and tocotrienol. However α-tocopherol acetate, and especially d,1-α-tocopherol acetate, is preferably used. The vitamin E and the edible triglyceride (when present) are preferably present in a weight ratio of 1:100 to 100:1, more preferably 20:80 to 98:2, still more preferably 75:25 to 95:5, especially 85:15 to 93:7 or vitamin E should provide 80-120% of its recommended daily allowance.

The emulsifying agent used in the compositions of the invention is preferably a phospholipid. However in place of, or in addition to, the phospholipid other fatty acid ester emulsifying agents may be used, e.g. esters of fatty acids (e.g. C₁₆₋₂₂, especially C₁₈ fatty acids) and polyhydric alcohols (especially C₆ alcohols) or polyoxyethylated derivatives thereof, in particular the span and tween non-ionic surfactants, especially polysorbate 80 (i.e. Tween^{®} 80), ethoxylated/propoxylated block polymers, for example, poloxamers, alkylpolyglycosides, and polyacrylic acid polymers, for example, Carbopol and Pemulen type emulsifiers. Nonetheless, while such emulsifiers, in particular polysorbate 80, have found use in the pharmaceutical and dietary supplement area, they are not generally preferred for use in foodstuffs and the use of phopholipids in the compositions of the invention is preferred.

The phospholipid used in the compositions of the invention is preferably a glycerophospholipid, a lysophospholipid or a sphingophospholipid, e.g. a sphingomyelin (SPH), cerebroside or ganglioside. Examples of glycerophospholipids include phosphatidic acids (PA), phosphatidylethanolamines (PE), phosphatidylcholines (PC), phosphatidyl-glycerophosphates, N-acyl-phosphatidyl-ethanolamines, phosphatidylserines (PS), phosphatidylinositols (PI), phosphatidylglycerols, diphosphatidylglycerols and plasmalogens. Examples of lysophospholipids include lysophosphatidylcholines, lysophosphotidylethanolamines, lysophosphatidylinositols, lysophosphatidylserines, lysophosphatidylglycerols, lysophosphatidylglycerophosphates, lysodiphosphatidylglycerols, lyso-N-acylphosphatidylethanolamines and lysophosphatidic acids, Glycerophospholipids, for example phosphatidylcholines, are particularly preferred. The phospholipid may be naturally occurring, synthetic or semisynthetic; however avian egg phospholipids or plant-derived natural phospholipids such as lecithins are especially preferred, e.g. soyabean, sunflower, rapeseed, corn or peanut lecithins. By semisynthetic phospholipids is meant a natural phospholipid which has been subjected to chemical modification, e.g. hydrolysis, for example enzymatic hydrolysis with phospholipases such as phospholipase A₁, A₂, B, C, or D, especially phospholipase A₂. Single phospholipids or combinations of two or more phospholipids may be used. Plant derived lecithins generally contain a mixture of phospholipids, e.g. PC together with one or more of PE, PI, PS, PA and SPH. One example of a particularly suitable commercially available food grade phospholipid is Emultop (available from Lucas Meyer GmbH, Hamburg, DE), a deoiled, enzymatically hydrolysed, powdered soybean lecithin enriched with lysophospholipids. Lecithins are also particularly preferred for use as the phospholipids due to their tocopherol content and inherent antioxidative properties.

The emulsifying agent, e.g. the phospholipids or fatty acid ester emulsifiers, are preferably used in the preparation of both the aqueous and oil phases before subsequent emulsification. The weight ratio of emulsifying agent to the total oil phase is preferably 1:3 to 1:25, more preferably 1:5 to 1:20, more preferably 1:7 to 1:15, especially 1:8 to 1:12.

Alternatively, the weight ratio of emulsifying agent to the total oil phase is 1:5 to 1:200, more preferably 1:10 to 1:100, especially 1:12 to 1:70.

It is thought that the phospholipid or fatty acid ester provides the oil droplets in the emulsion with an at least partial surface membrane which serves to promote stability both of the emulsion and of the vitamins, and/or non-vitamin lipophilic drugs dispersed in the droplets. The protection of the lipophilic vitamins and non-vitamin drugs may arise as a result of reduced oxygen diffusion across the oil-water interface of the emulsion droplets and desirably the concentration of the vitamins other than vitamin E in the oil phase is relatively low in order to have a low ratio between their in-oil concentration and the oil-water interface surface area.

The lipophilic or hydrophilic vitamins, or non-vitamin drugs, or other agents (i.e. minerals) present in the composition, may be incorporated into particles or droplets (e.g. droplets of deoxygenated water solution) within the oil phase droplets in the emulsion. The particles or droplets may have a small diameter e.g. 1 to 1000 nm, preferably 5 to 800 nm, especially 10 to 600 nm. The particles or droplets would therefore be protected from exposure to oxygen. Thus, a water-in-oil-in-water emulsion is also considered in the emulsion of the invention.

The lipophilic vitamins suitable for use in the invention include vitamin E, vitamin A, vitamin K and/or vitamin D, especially vitamin A, vitamin D and vitamin E, particularly vitamin E. However, any one of these vitamins, or any combination of the foregoing, would be suitable for use in the composition of the invention.

The vitamin D used in the compositions of the invention may be in any one of its various active forms including derivatives, analogues, metabolites and bioprecursors, e.g. cholecalciferol (vitamin D₃), ergocalciferol (vitamin D₂), 1α,25-dihydroxy vitamin D, 25-hydroxy vitamin D, 1α-hydroxy vitamin D, etc. Ergocalciferol and, even more so, cholecalciferol are preferred. Vitamin D₃ is readily available commercially in an edible oil base, e.g. from Roche. Such forms may include edible triglycerides and it should be noted that the total quantity of edible triglycerides in the composition may include some deriving from the vitamin D mix.

The vitamin A used in the compositions of the invention may be used in any of its various active forms including analogues, derivatives, metabolites and bioprecursors e.g. retinols, esters of retinol, dehydroretinol and beta-carotene. Most preferred in the composition of the invention is retinol.

The vitamin K used in the compositions of the invention may be used in any of its various active forms including analogues, derivatives, metabolites and bioprecursors, e.g. phytonadione, menaquinone and menadione.

The water-soluble vitamins suitable for use in the composition of the invention include thiamine, riboflavin, niacin, nicotinamide, Vitamin B₆ group, biotin, pantothenic acid, folic acid, pyridoxine, pyridoxal, pyridoxamine, inositol, vitamin B₁₂, choline and/or ascorbic acid. Any one of these vitamins, or any combination of the foregoing, may be used in the composition of the invention.

Nicotinamide (also known as a B complex vitamin) may be used in any available active forms, including analogues, derivatives, metabolites and precursors.

Thiamine (vitamin B₁) may be used in any available active form, including analogues, derivatives, metabolites and precursors. Preferred forms include thiamine pyrophosphate, thiamine hydrochloride and thiamine mononitrate.

Riboflavine (vitamin B₂) may be used in any available active form, including analogues, derivatives, metabolites and precursors. Preferred forms include riboflavine, riboflavine 5' phosphate and riboflavine 5' phosphate sodium.

Pantothenic acid (a B complex vitamin) may be used in any available active form, including analogues, derivatives, metabolites, precursors and as a salt. As a salt, dexpanthenol is preferred.

Pyridoxine (a vitamin B₆) may be used in any available active form, including analogues, derivatives, metabolites and precursors. (Other B₆ vitamins include pyridoxal and pyridoxamine, which can also be used in the composition of the invention.)

Folic acid (a B complex vitamin) may be used in any available active form, including analogues, derivatives, metabolites and precursors.

Ascorbic acid (vitamin C) may be used in any available active form, including analogues, derivatives, metabolites, precursors and as a salt, especially sodium, potassium or calcium ascorbate.

Certain of the vitamins have relatively low water solubility, e.g. riboflavin and folic acid, and these may be included in the compositions of the invention in dispersed rather than fully dissolved form.

Preferably the composition of the invention will contain vitamins and/or minerals in the range of 15 to 500% of the Recommended Daily Allowance (RDA), preferably 30 to 200% RDA, especially 80 to 120% RDA per dose. The RDAs as specified in the Council Directive of 24 September 1990 on nutrition labelling for foodstuffs (90/496/EEC) are as follows:

| | |
|---|---|
| Vitamin A: | 800 µg |
| Vitamin D: | 5 µg |
| Vitamin E: | 10 mg |
| Vitamin C: | 60 mg |
| Thiamin: | 1.4 mg |
| Riboflavin: | 1.6 mg |
| Niacin: | 18 mg |
| Vitamin B₆: | 2 mg |
| Folic acid: | 200 µg |
| Vitamin B₁₂ | 2 µg |
| Biotin: | 0.15 mg |
| Pantothenic acid: | 6 mg |
| Vitamin K: | 50 µg |

The recommended daily amounts of vitamin B₁₂ and vitamin K are taken from "Nordic guidelines for intake of nutrients, 1996".

An overage of vitamin may be used in the composition of the invention, in order to compensate for any degradation. However, the vitamins used in the composition of the invention are relatively stable, and therefore only small overages, in the range of 0 to 25%, preferably 0 to 15%, more preferably 0 to 10%, e.g. 5 to 10% may be used.

An overage of 10% of vitamins A and D will ensure a shelf-life of 18 months at room temperature. An overage of 5% of vitamin E (DL-α-tocopheryl acetate) ensures the same shelf-life. The following overages may be used for the water-soluble vitamins: thiamine nitrate (10%), nicotinamide (5%), ascorbic acid (25%), pyridoxine hydrochloride (5%), dexpanthenol (10%), vitamin B₁₂ (20%), riboflavine (5%) and folic acid (20%).

Desirably, the compositions of the invention contain: optionally 120 µg to 4000 µg, preferably 640 µg to 960 *µ*g vitamin A; optionally 0.75 *µ*g to 25 *µ*g, preferably 4 *µ*g to 6 *µ*g vitamin D; optionally 9 to 300 mg, preferably 48 to 72 mg vitamin C; optionally 0.21 mg to 7 mg, preferably 1.12 mg to 1.68 mg Thiamin (vitamin B1); optionally 0.24 mg to 8 mg, preferably 1.28 mg to 1.92 mg Riboflavin (vitamin B₂); optionally 2.7 mg to 90 mg, preferably 14.4 to 21.6 mg Niacin (vitamin B₃), optionally 0.3 to 10 mg preferably 1.6 mg to 2.4 mg Pyridoxine (vitamin B₆); optionally 30 µg to 1000 µg preferably 160 µg to 240 µg folic acid (vitamin B₉); optionally 0.6 µg to 6 µg, preferably 1.6 to 2.4 µg, vitamin B₁₂; optionally 0.225 mg to 0.75 mg, preferably 0.12 to 0.18 mg biotin; optionally 0.9 mg to 30 mg, preferably 4.8 mg to 7.2 mg pantothenic acid (vitamin B₅); and/or 7.5 µg to 250 µg, preferably 40 µg to 60 µg vitamin K.

Besides the lipophilic vitamins/non-vitamin drugs, the optional water-soluble vitamins, the emulsifying agent (e.g. phospholipid), the gelling agent and the thickener and water, the compositions according to the invention may, and indeed generally will, contain other physiologically tolerable components, for example sweeteners, starches, antioxidants, isoflavones, beta-carotene, lycopene, soluble and insoluble fibre, minerals (e.g. zinc or iron), colouring agents, pH modifiers (e.g. buffering agents or acidifiers, for example citric acid, lactic acid, malic acid, etc.) preservatives (e.g. benzoates and sorbates), flavours, etc.

The compositions of the invention contain a viscosity modifier, i.e. a material which increases the viscosity of the aqueous phase, most preferably the combination of a thickener (for example, a gum) and agar agar as a gelling agent, for example the combination of agar agar and an edible gum such as locust bean gum, guar gum, xanthan gum, gum arabic, or gum tragacanth. Other examples of thickening agents include cellulose derivatives, for example, methyl cellulose, carboxymethylcellulose, hydroxypropylcellulose, methyl hydroxypropylcellulose and hydroxypropylmethylcellulose and modified starches based on maize, waxy maize, potato, wheat, rice and tapioca. The gelling agent and the thickener combination will generally be used at total concentrations of 0.01 to 5% w/w of the total liquid emulsion composition, more preferably 0.05 to 3% w/w, especially 0.1 to 1.5% w/w. The gelling agent and the thickener combination serve to enhance the physical stability of the emulsion. A gelling agent is defined as being a material capable of forming a gel on dissolution in water. Gelling agents include agar agar alginates, more specifically Na-alginate, K-alginate, NH₄-alginate, Mg-alginate or Ca-alginate, propylene glycol alginate, iota-carrageenan gellan gum, more specifically high acyl gellan gum or low acyl gellan gum, pectins, more specifically high methoxyl pectin or low methoxyl pectin. The gelling agent is preferably used at a concentration of 0.02 to 1% w/w of the total composition, more preferably 0.03 to 0.4% w/w, especially 0.04 to 0.3% w/w. The gelling agent is agar agar and this is especially preferably used together with one or more edible gums, e.g. locust bean gum and guar gum. The thickener is preferably used at a concentration of 0.05 to 1.5% by weight of the total composition.

The compositions of the invention are intended for oral ingestion. For oral ingestion, the compositions desirably contain sweeteners and flavours to enhance their acceptability to the consumer. The sweeteners used may be natural sweeteners, e.g. mono, di and polysacchrides, for example sucrose, fructose, fructooligosaccharides (oligofructoses), glucose, glucose syrup, invert sugar, maltodextrins or sugar alcohols such as sorbitol, sorbitol syrup, maltitol, maltitol syrup, lactitol, mannitol, xylitol, isomalt, etc., or artificial sweeteners. Examples of intense artificial sweeteners include aspartame, acesulfam K, neohesperidine dihydrochalcone, thaumatin, saccharin, saccharin salts (i.e. sodium saccharin) and cyclamates and cyclamic acid. A single sweetener or a combination of two or more sweeteners may be used. Preferred natural sweeteners are sugar and fructose conveniently used as syrups with 70% solids (on drying), likewise sorbitol as a 70% syrup, and fructooligosaccharides. A particularly preferred combination is aspartame and acesulfam K, e.g. in a 2:1 to 1:2 weight ratio, especially a 0.9:1 to 1:0.9 ratio.

Especially preferred a combination of aspartame, acesulfam and inulin and/or fructooligosaccherides is used as the combination has a synergistic taste effect, relatively effectively mimicking the sweetening effect of sugar and masking any harsh taste of the artificial sweeteners. Fructooligosaccharides can be obtained by partial hydrolysis of inulin and are available under the trade name Raftilose from Orafti SA, Tienen, Belgium, which firm also supplies inulins under the trade name Raftiline. Fructooligosaccharides are also available under the trade name Actilight from Beghin-Meiji Industries, Neuilly-sur-Seine, France. Generally the inulin or fructooligosaccharide will be used in 100-5000 parts by weight per 2 parts by weight of aspartame and acesulfam.

The content of sweetener in the compositions of the invention will depend upon the particular sweeteners used and on whether the composition is to be diluted before consumption. Thus the sweetener content will be chosen so as to give a pleasant sweetness on consumption. Typically the sweetener content will be 0.05 to 1% w/w where intense artificial sweeteners are used, e.g. about 0.1 to 0.3% w/w. Where natural sweeteners (e.g. invert sugar or fructose) are used, they can typically make up 20-50% w/w, more preferably 30-50% w/w of the overall composition on a dry solids basis.

For compositions intended for adolescents and children not in need of low calorie products, natural sweeteners (e.g. sugar alcohols) and non-carcinogenic sweeteners may be preferred over artificial sweeteners. However, for products intended for calorie-conscious adults, artificial sweeteners may be preferred.

Examples of flavouring agents useful in the compositions of the invention include fruit (e.g. pineapple or citrus) concentrates and concentrated aqueous or non-aqueous flavours such as flavour oils, e.g. citrus oils, for example cold pressed orange oil (B.P.). Orange concentrate, e.g. 65 Brix orange concentrate is particularly suitable. The flavouring agent will be used at a concentration sufficient to give the composition, optionally after dilution, a pleasant taste. By way of example 65 Brix orange concentrate may be used at a concentration of 1 to 20% w/w relative to the total emulsion, preferably 2 to 15% w/w. Alternatively cold pressed orange oil BP may be used at a concentration of 0.04 to 0.3% w/w, preferably 0.06 to 0.2% w/w, relatively to the total emulsion.

It should be recognised that the use of flavours or acidifiers which are soluble in the aqueous phase (e.g. fruit concentrates or citric acid) may affect the solubility of the vitamins in that phase and that it may be necessary in such cases to dilute the aqueous phase to prevent precipitation. Accordingly, acidifiers such as lactic acid and water-insoluble flavours such as citrus oils or water-soluble flavours such as strawberry, raspberry, passion fruit, exotic fruit, peach and apricot flavours and other non-citrus flavours such as pineapple concentrate are preferred.

Where a flavour oil is used, it may be dispersed in the oil phase together with the lipophilic vitamins, or non-vitamin lipophilic drugs, or a combination thereof or alternatively and preferably the flavour and the lipophilic vitamins and/or non-vitamin drugs are separately dispersed in the overall emulsion - in this way any effect of the flavour oil on vitamin stability may be minimized. In these circumstances a phospholipid or another emulsifier is preferably dissolved in the flavour oil and two oil phases, one containing flavour oil and the other containing the lipophilic vitamins and/or non-vitamin lipophilic drugs are intensively mixed with the aqueous phase. This can be done separately (with the two emulsions then being mixed together) or sequentially (with one oil phase, generally the vitamin phase, being intensively mixed with some or all of the aqueous phase and the second oil phase then being intensively mixed with the resulting emulsion (optionally after dilution of this emulsion).

In general the low density of the oils in the disperse phase of an emulsion is one of the major causes of physical instability with a resultant creaming of the emulsion. The density of orange oil, BP is in the range of 0.85 0.88 g/ml and thus much smaller than the density of the bulk aqueous phase which in this case in the range of 1.16 to 1.23 g/ml, for example, 1.16 to 1.19 g/ml. Selecting the appropriate gelling and thickening system thus becomes crucial in order to prevent the separation of the two phases. The gelling and thickening system employed for the formulations in this invention is unique in this respect. This system exhibits gel-sol-gel properties when exposed to shearing stress. The aqueous phase has a gel structure at rest which lock the emulsion droplets in a three-dimensional network of gelling and thickening agents. The emulsion will however flow easily when exposed to only slight stress like turning the bottle upside down or shaking it. The emulsion will gradually loose viscosity in the mouth and thus will not be perceived as slimy by the consumer.

Suitable preservatives for use in the compositions of the invention include food grade preservatives, for example the potassium and sodium salts of sorbic, benzoic and parahydroxybenzoic acids. Potassium sorbate is especially preferred. The preservative will generally be used at concentrations of 0.05 to 1.5% w/w relative to the total emulsion, preferably 0.1 to 0.3 w/w.

As a colouring agent, beta-carotene may for example be used. Beta-carotene gives the emulsion an orange colour which matches the orange flavour where an orange flavour is used. Beta-carotene as an oily suspension (beta-carotene 30% FS from Roche) or cold-water soluble beta-carotene (beta-carotene 7% CWS from Roche) may be used.

Acidifying agents, e.g. lactic or malic acid, may be included in the compositions of the invention. Lactic acid, available in 80% solution as Purac 80 from Purac biochem bv is preferred. Preferably the pH of the emulsion should be adjusted to below 6, more preferably below 5, e.g. in the range 3 to 5. Where fructooligosaccharides are used in the compositions of the invention, the pH is desirably kept above 4 to avoid hydrolysis.

In a preferred embodiment of the invention, a physiologically tolerable inorganic compound of nanometer size (e.g. 1 to 1000 nm, preferably 5 to 800 nm, especially 10 to 600 nm) is added to the disperse phase of the emulsion in order to further stabilize the oil-water emulsion. The inorganic compound used is desirably of higher density than the oil phase, and preferably of higher density than the aqueous phase too. Suitable inorganic compounds include calcium salts, i.e. calcium carbonate, calcium lactate, calcium gluconate, calcium citrate, calcium malate, calcium hydroxide and calcium phosphate, preferably calcium carbonate. Other suitable compounds include sodium salts, magnesium salts and zinc salts. Preferably, the inorganic compound is calcium carbonate, which is commercially available in a nanometer size. The inorganic compound thus increases the density of the oil phase, and may if desired be used in quantities sufficient to form an isodense oil-in-water emulsion.

Thus viewed from a further aspect the invention provides a liquid emulsion composition having a continuous aqueous phase containing a gelling agent and a thickener and optionally a physiologically tolerable amount of at least one water soluble vitamin and/or non-vitamin drug, and a discontinuous oil phase comprising at least one lipophilic vitamin and optionally a non-vitamin drug and optionally an edible triglyceride, said emulsion composition further containing at least one emulsifying agent, preferably one selected from edible phospholipids and fatty acid esters, said oil phase comprising at least one of (i) droplets of a discontinuous aqueous phase containing a physiologically active of beneficial compound dissolved therein, (ii) an inorganic particulate, and (iii) a non-vitamin lipophilic drug compound.

The compositions of the invention are oil-in-water emulsions, preferably with a narrow oil (triglyceride) droplet size distribution with the weight average droplet size (i.e. diameter) (measured for example by light microscopy and comparison with a 1 to 10 µm scale) in the range 1 to 5 µm, more preferably 1 to 4 µm, even more preferably 2 to 4 µm. Emulsification is preferably effected in such a way as to have only a small oversize fraction of droplets, i.e. droplets above 5 µm in diameter. This may be achieved by mixing the aqueous phase and the oil phase using a high intensity mixer, a high speed colloid mill (Koruma, Ytron, Siverson or Ystral), or a high pressure homogenizer (micro fluidiser) for example a high shear rotor stator mixer, available for example from Ystral GmbH, Dottingen, DE. One example of a suitable mixer is the Diax 600 with a 20G or 20F shaft. An in-line dispersion chamber (e.g. Diax 600, type 22/Z) is preferably used as this can ensure that little or no air is introduced into the emulsion.

It may be more efficient to create an emulsion using only part of the aqueous phase and then to add the emulsion to the remaining portion or portions of the aqueous phase.

In the process of the invention, the gelling agent, agar agar has to be heated in an aqueous medium to above its gel point, for example, 95 to 100°C , in order for it to dissolve. However, the vitamins should not be exposed to a temperature higher than 40°C, more preferably not to a temperature above 30°C. Cooling of the liquid agar agar solution may be effected by the addition of a further solution, such as sorbitol and/or the thickener solution (guar gum and/or locust bean gum) solutions. During cooling of the agar, thickening agents and bulk sweetener solution, care must be taken to prevent gel formation. The agar agar solution has to be cooled through around 32 to 28°C using gentle stirring. Stirring is such that the viscosity does not exceed 3000 mPa.s (cps) preferably 2500 mPa.s (cps), especially 1500 mPa.s (cps), on cooling to 25°C.

The overall volume of water used is preferably kept to the minimum required to keep the vitamins (when present) stably in solution. The proportions of this water used to prepare the different aqueous compositions will generally be selected to be at least the minimum required to produce compositions which can be poured and mixed together, the total desired water content can be made up by addition of water or of aqueous solutions of further components. In this way evaporation losses can be compensated for.

Optionally production and handling is carried out under an inert (e.g. nitrogen or inert (e.g. noble gas)) atmosphere, under a partial vacuum or with nitrogen injection so as to minimize the oxygen contact with the vitamin D, vitamin A and vitamin E. Alternatively oxygen contact may be reduced by preparing the lipophilic vitamin composition and emulsifying this with the thickener solution under an inert atmosphere.

For preparation of emulsions of the inventions, use of a high shear rotor stator mixer or an in-line high speed dispersion rotor stator mixer is preferred.

The containers used may be single dose containers, e.g. bottles, sachets, vials, etc; however multi-dose containers are preferred, e.g. 50 to 1000 mL bottles, preferably 500 mL bottles. If the containers are light transmitting, they are preferably brown-coloured, e.g. brown coloured PET. Before the containers are sealed, the head space above the emulsion may if desired be flushed with an oxygen-free gas, e.g. nitrogen.

As mentioned above, deaeration or nitrogen injection is optionally used during the preparation of the emulsion product to exclude oxygen.

Such emulsions may be used directly. Alternatively, such emulsions may conveniently be diluted 1 part with 5 parts by volume of diluent, e.g. tap water or mineral water, milk, fruit juice, or any other alcohol-free beverage. Where water is used the resultant diluted composition may be a clear liquid or a non-clear liquid with an acceptable flavour.

Doses of the composition of the invention may be taken between meals or with meals and are suitable for older people with reduced gastric acid secretion.

The compositions of the invention may be used in therapeutic or prophylactic treatment and this forms a further aspect of the invention. Viewed from this aspect the invention may be used in a method of treatment of a human or non-human mammal subject to combat conditions associated with vitamin deficiency (e.g. beri-beri, nyctalopia, megaloblastic haemopoiesis, pernicious anemia, hypoprothrombis anemia, pellegra, sprue, scurvy, rickets), said method comprising orally administering said subject a composition or supplement according to the invention.

It will be clear to a person skilled in the art that the compositions, processes and methods of the invention could be extended to the formulation of other food supplements. For example, the composition of the invention could be used to formulate multimineral compositions, combined multivitamin and mineral compositions. Thus, in a preferred embodiment, the compositions of the invention include multiminerals, such as zinc, iron, calcium, iron, iodine, magnesium and phosphorus. Preferably, the minerals are present as inorganic and/or organic salts in the aqueous phase of the composition, for example as calcium lactate, zinc sulphate, potassium iodide, ferrous sulphate and/or magnesium carbonate. Suitable compounds are well known in the art.

Preferably, the minerals are complexed with suitable chelating agents, such as aminopolycarboxylic acids (e.g. EDTA or DTPA) to prevent oxidation of the vitamins in the aqueous phase. Soluble minerals salts may be used in equimolar concentrations with pyrophosphate to give pyrophosphate complexes.

The minerals are optionally present at 15 to 500% RDA, preferably 80 to 120% RDA, as set by the Council Directive (supra).

| | |
|---|---|
| Calcium: | 800 mg |
| Phosphorus: | 800 mg |
| Iron: | 14 mg |
| Magnesium: | 300 mg |
| Zinc: | 15 mg |
| Iodine: | 150 µg |
| Copper: | 2 mg |
| Manganese: | 1 mg |
| Chromium: | 50 µg |
| Selenium: | 40 µg |
| Molybdenum: | 150 µg |

In a further preferred embodiment of the invention, non-vitamin lipophilic drugs can be included in the oil phase of the emulsion and/or hydrophilic non-vitamin drugs can be included in the aqueous phase of the emulsion, together with the lipophilic and optionally the hydrophilic vitamins.

Viewed from this aspect the invention provides a pharmaceutical composition in emulsion form comprising a discontinuous oil phase containing an edible oil with optionally dissolved or dispersed therein a non-vitamin drug compound, preferably a lipophilic non-vitamin drug compound or compound mixture, and a continuous aqueous phase containing a gelling agent and a thickener (e.g. guar and/or locust bean gum) and an emulsifying agent (preferably selected from edible phospholipids and fatty acid esters), and optionally a hydrophilic non-vitamin drug compound or compound mixture.

Presentation or formulation of the lipophilic non-vitamin drug compound in this emulsion technology may have the advantage of increasing the bioavailability, particularly oral bioavailability, of a non-vitamin drug, especially a poorly water-soluble drug, due to presentation in an easily-absorbed form such as a dissolved and/or solubilised form. From the gastrointestinal tract the lipophilic non-vitamin drug might be either absorbed the common way into the portal blood or by lympatic absorption. Lympatic absorption is possible because fatty acids and bile salts are present during digestion. The lipophilic non-vitamin drug substance may be absorbed in association with the fatty acid/bile acid micellar phase and therefore be associated in the formation of chylomicrons which are transported into the lymphatic circulation. Through absorption as fat globules into the lymphatic system, the lipophilic non-vitamin drug does not go through any intermediate dissolution stage which is often a rate limiting step for absorption of poorly soluble and lipophilic non-vitamin drugs.

The bioavailability of drugs which are exposed to first pass metabolism in the gastro-intestinal tract or in the liver may be increased due to absorption via the lymphatic system. A further advantage due to the facilitated absorption may be an increase of the absorption rate and thus rapid onset of clinical effect.

Furthermore, the incorporation of the non-vitamin drug substance in the lipid phase of the emulsion might protect the drug molecule from the acid environment in the stomach, and thereby protect the drug from degeneration in the gastric fluid, resulting in an increased bioavailability.

The term drug compound as used herein does not include essential nutrients or their bioprecursors, i.e. vitamins, triglycerides, etc.

Examples of lipophilic non-vitamin drug candidates for the disperse phase and hydrophilic non-vitamin drugs for the aqueous phase particularly relevant for peroral administration are analgesics such as synthetic opioids (e.g. fentanyl, alentanil, sufentanil) and non-steroidal anti-inflammatory drugs (e.g. naproxen, penylbutazone, acetylsalicylic acid). Liquid formulations of anticonvulsants such as carbamazepine, phenytoin and benzodiazepines (e.g. diazepam, clonazepam, midazolam, and nitrazepam), adrenergica (e.g. loratadin and pseudoephedrin, acrivastine and pseudoephdrine) expectorantia/mucolytica (e.g. bromhexin, ammonium chloride, acetyl cysteine, carbocisteine, cocillana, creosote, domidol, guiaphenesin, senega root, terpin hydrate) antitussives (e.g. codein, dextromethorphan, noscapin, ethylmorphine, acetyldihydrocodeine, benzonatate, chlophedianol, clobutinol, dimemorfan, drotebanol, levopropoxyphene, morclofone, thebacon, ziperprol) antihistamines (e.g. acrivastin, cetirizin, ebastine, dexchlorpheniramin, dexbromopheniramin, flunarizine, pizotifen, trimethobenzamide), anti-infectives including antibiotics like penicillins, cephalosporins, betalactam antibiotics, aminoglycosides, tetracyclines, chloramphenicol, macrolides, clindamycin, spectinomycin, polymyxin B, colistin, vancomycin, bacitracin, isoniacid, rifampin, ethambutol, streptomycin, pyrazinamide, ethionamide, cycloserine and aminosalicylic acid, non-opioid analgesics (e.g. paracetamol, acetylsalicylic acid, ibuprofen) are also of value, especially in the treatment of children and elderly. The absorption of a number of other biologically active lipophilic and/or poorly soluble substances may be improved by use of the emulsion technology. Examples of such substances are: Corticosteroids (e.g. hydrocortisone, prednisone, prednisolone), androgens (e.g. testosterone, nandrolone,), progestogens (e.g progesterone, norethisterone, danazol), oestrogens (e.g. megastol, ethinyloestradiol, mestranol), drugs for treatment of Parkinson's disease (e.g. levodopa, carbidopa), anticonvulsants (e.g. carbamazepine, phenytoin), antifungal agents (e.g. griseofulvin, clotrimazole), antibacterials (e.g. nitrofurantoin, sulphapyridine, tetracycline, ceftrioxane), antivirals (e.g. zidovudine), tricyclic antidepressants (e.g. imipramine, amitriptyline) immunosuppressants (e.g cyclosporine A, dihydrocyclosporine D), antineoplastic agents (e.g. 5-fluorouracil, chlorambucil, mercaptopurine, fenretinide), antimalarials (e.g. halofantrine), vasodilators (e.g. cyclandelate), anxientiolytica (e.g. gepirone), antihistamines (e.g. repirinast, cinnarizine, fexofenadine), lipid regulation agents (e.g. probucol), anticoagulantia (e.g. dicuomarol), beta-blockers (e.g. propranolol), therapeutic vitamines (e.g. menatetrenone), antihypertensives (e.g. felodipine, nifedipine, penclomedine), anti protozoal agents (e.g. atovaquone), diuretics (e.g. spironolactone), opioid agonists (e.g. oxycodone), antidepressant (e.g. vanoxerine), antidiabetic agents (e.g. glibenclamide).

Preferred lipophilic drug candidates are Probucol, Diazepam, Danazol, Halofantrine and Cyclosporin A.

Agents for the control of gastric acidity and treatment of peptic ulcers such as cimetidine, ranitidine, famotidine, omeprazole and lansoprazole are also suitable for use in the compositions of the invention.

Combination products can easily be formulated where the lipophilic non-vitamin drug is contained in the disperse phase while a hydrophilic non-vitamin drug is dissolved in the continuous aqueous phase. Cough and cold formulations are typical common combination products where a cough suppressant is combined with one or two analgesics. Preferred cough and cold drug candidates are dextomethorphan, bromhexin and acetylcystein.

High dosages of drugs can be given due to the flexibility in the dosage volume where up to 10 ml can be given as a single dosage. The possible dosage for a lipophilic non-vitamin drug will depend on the solubility in the disperse phase but may be as high as 500 mg in the case where the drug is a fluid lipid itself. More typically the lipophilic drug will be in the range of sub-micron amounts up to 100 mg. The content of the hydrophilic non-vitamin drug contained in the continuous phase will likewise depend on the aqueous solubility but the available volume for dissolution is larger with a resultant possibility of inclusion of a high amount of active per dosage.

Special emulsion systems like multiple emulsions can also be formulated. A water-in-oil-in-water emulsion (w/o/w) as discussed previously may be suitable where an effective protection of the active ingredient is sought. Peptide hormones like insulin is an example of such case where the hormone needs to be protected from the proteolytic enzymes in the gastro-intestinal during the absorption process.

Viewed from a further aspect, the invention provides a pharmaceutical emulsion according to the invention, said emulsion comprising at least one lipophilic and/or hydrophilic non-vitamin drug, wherein the disperse phase of the emulsion includes a physiologically tolerable inorganic compound of nanometer size (e.g. 1 to 1000 nm, preferably 5 to 800 nm, especially 10 to 600 nm) to further stabilize the oil-water or water-in-oil-in-water emulsion. The inorganic compound used is desirably of higher density than the oil phase, and preferably of higher density than the aqueous phase too. Suitable inorganic compounds include calcium salts, i.e. calcium carbonate, calcium lactate, calcium gluconate, calcium citrate, calcium malate, calcium hydroxide and calcium phosphate, preferably calcium carbonate. Other suitable compounds include sodium salts, magnesium salts and zinc salts. Preferably, the inorganic compound is calcium carbonate, which is commercially available in a nanometer size. The inorganic compound thus increases the density of the oil phase, and may if desired be used in quantities sufficient to form an isodense oil-in-water emulsion.

The invention will now be described further with reference to the following Examples :

### EXAMPLE 1

### Preparation of a liquid syrup

### Ingredients:

| | | |
|---|---|---|
| Vitamin oil mixture: (6.5g in total) | DL-α-tocopherol acetate | 4700 mg |
| | Vitamin A palmitate | 539 mg |
| | Cholecalciferol concentrate | 165 mg |
| | Lecithin | 500 mg |
| | Medium chain (C₈-C₁₂) tryglycerides | 596 mg |
| | | |
| Vitamin powder mixture: | Nicotinamide | 6.3 mg |
| (10g in total) | Thiamine nitrate | 612 mg |
| | Riboflavine | 550 mg |
| | Pyridoxine hydrochloride | 765 mg |
| | Folic acid | 45 mg |
| | Sorbitol powder | 1728 mg |
| | | |
| Dexpanthenol | | 2 g |
| Ascorbic acid | | 28 g |
| Potassium sorbate: | | 3.5 g |
| Sorbitol 70% (non-crystallising): | | 3.55 kg |
| Agar agar: | | 4 g |
| Sorbitol powder: | | 54 g |
| Galactomannan mixture: | | 17 g |
| Citric acid monohydrate: | | 20 g |
| Orange oil: | | 3.4 g |
| Lecithin: | | 0.3 g |
| Purified water: | | 2190 g |

A first batch of water is heated to a temperature of 60°C, to which agar agar is added and dispersed with high speed mixer. The mixture is heated to 95°C to dissolve the agar agar to produce liquid (A). Liquid (A) is maintained above the gel point (28-35°C), e.g. at 50°C. A second batch of water is heated to a temperature of 70°C. A 65:35 ratio locust bean gum:guar gum mixture is added and dispersed with a high speed mixer to produce liquid (B). Liquid (B) is maintained at a temperature above the gel point of liquid (A), e.g. at 50°C. A fraction, e.g. 5-10%, of liquid (B) is removed and cooled to about 30°C and diluted with water to reduce viscosity to a level suitable for emulsification. The resultant liquid is liquid (C). The remainder of liquid (B) with liquid (A) are combined and the resulting liquid, liquid (D) is maintained above the gel point, e.g. 50°C. Liquid sorbitol is added and the temperature is gradually brought down to 30-35°C. Dexpanthenol is gently heated in a water bath so that it can be easily transferred and then added to the main liquid (D). Lecithin is added to DL-α-tocopherol acetate and heated to 50°C to dissolve the lecithin and cooled to about 30°C. The lipophilic vitamins are added (e.g. vitamin A, vitamin D, betacarotene and vitamin K) to produce liquid (E). Citrus oil (e.g. orange oil) is mixed with lecithin and warmed slightly, e.g. to about 30°C to dissolve the lecithin. The resultant liquid is liquid (F). Liquid (E) is added slowly to liquid (C) in a high intensity mixer to produce a pre-emulsion. Liquid (F) is mixed also with a high intensity mixer (i.e. a Diax 600 dispersion machine with a 20G shaft). The resultant pre-emulsion is liquid (G). The vitamin powder mixture (i.e. nicotinamide, thiamine mononitrate, riboflavine, pyridoxine hydrochloride, folic acid and sorbitol) is mixed together with ascorbic acid and citric acid monohydrate in a batch of water in a high intensity mixer with a dispersion shaft to produce liquid (H). The main liquid (D) is cooled to about 25°C and liquid (G) and liquid (H) are added. The mixtures are homogenized for 2 minutes in a high intensity mixer with a dispersion shaft taking care not to introduce air into the mixture. The resultant mixture is filled into bottles and optionally sealed under nitrogen.

The vitamin emulsion is a homogeneous and smooth-flowing yellow or orange coloured syrup with a fresh citrus taste. The syrup has a pH of 3.0 to 3.6, a viscosity of 300 to 1000 mPa.s (cps) at 20°C and a density of 1.16 to 1.23 g/ml at 20°C. The diameter of the bulk of the oil droplets in the emulsion is between 2 to 5 µm.

The investigation of viscosity behaviour for the vitamin emulsion was carried out using a stress-controlled instrument called DSR200 from Rheometric Scientific. The first test used was a thixotropy loop test where after mounting, the sample was left for 10 minutes. Then a clockwise rotation of the upper fixture was achieved by ramping the stress from zero to 40 Pa in 10 minutes and then down to 0 Pa in another 10 minutes. After a rest period of 40 minutes the same sequence with a counter-clockwise rotation was performed.

The second test used was a step stress test where a sequence of stresses was programmed, first in increasing order and then in decreasing order. Each stress was kept constant for a given period. Stresses were applied in the range of 0.06 to 40 Pa in an increasing and descending step-wise fashion.

Results from test one showed that the reproducibility of the clockwise and counterclockwise were good. Only very little hysteresis is seen in ramping the stresses up and down except at the smallest stress values. Therefore the sample shows a thixotropic behaviour only at low stress levels. At higher stress levels (above 30 Pa) structure within the fluid is broken down. A characteristic shear thinning behaviour was evident for the sample.

Results from test two showed that for applied stresses lower than 0.8 Pa (or rates less than 1 s⁻¹) the rotational speed was fluctuating erratically, thus indicating structural changes within the sample as the sample was sheared.

The rheological characterisation showed the presence of thixotropy or a time-dependent decrease in viscosity at low shear stress which is reversible. In other words the emulsion exhibits gel-sol-gel properties. The second test showed the presence of structural rearrangements at small stress levels and the possibility of yield behaviour.

These findings present an explanation to the excellent physical stability of the emulsion and the presence of thixotropy at very low shear stresses (e.g. the emulsion can be poured after shaking the bottle lightly).

### EXAMPLE 2

### Chemical Stability

A stability study was carried out in order to investigate the chemical stability of the liquid multivitamin product. The product was manufactured according to the protocol set out in Example 1. Two production batches were prepared and subjected to a stability study. The following sampling times were used:
20°C/ambient 0, 3, 6, 12 and 18 months Relative Humidity (RH):

The following results for the vitamin contents after 18 months were obtained for the two production batches respectively at 20°C/ambient RH:

| Vitamin | Declared | Overage | Nominal | Initial | Recovery | 18 months | Degraded |
|---|---|---|---|---|---|---|---|
| Retinol (µg/10 ml) | 500 | 10% | 550 | 623, 628 | 113% | 518, 525 | 16% |
| Cholecalciferol (µg/10 ml) | 7.5 | 10% | 8.25 | 8.0, 8.1 | 97.6% | 7.3, 7.3 | 10% |
| D-α-tocopherol (mg/10 ml) | 6.0 | 5% | 6.30 | 6.2,6.3 | 99.2% | 6.1,6.2 | 2% |
| Nicotinamide (mg/10 ml) | 12 | 5% | 12.6 | 12.2, 12.3 | 97.2% | 11.6,11.9 | 4% |
| Thiamine (mg/10 ml) | 0.9 | 10% | 0.99 | 0.96, 0.97 | 97.5% | 0.88,0.89 | 8% |
| Riboflavine (mg/10 ml) | 1.0 | 10% | 1.10 | 1.05, 1.06 | 95.9% | 1.08, 1.09 | +2% |
| Pantothenic acid (mg/10 ml) | 4.0 | 10% | 4.40 | 4.3,4.4 | 98.9% | 3.6,3.6 | 16% |
| Ascorbic acid (mg/10 ml) | 45 | 25% | 56.3 | 54.4,55.3 | 97.4% | 42.8,43.6 | 20% |
| Pyridoxine (mg/10 ml) | 1.2 | 5% | 1.26 | 1.21, 1.22 | 96.4% | 1.25, 1.24 | +2% |
| Folic acid (µg/10 ml) | 75 | 20% | 90.0 | 92.1, 92.2 | 102.4% | 71.4, 72.5 | 21% |

The initial value for the analysis of retinol is somewhat high which is due to an initial high value of 106% of nominal value in the raw material.

The products have been analyzed with respect to all the vitamins, flavour, appearance, pH, total viable count, test for E.Coli, test for efficacy of antimicrobial preservation, potassium sorbate, viscosity, density and microscopic picture (droplet size of disperse phase).

The initial analysis of the two batches gave vitamin contents very near to the nominal amounts which indicate that there are no detectable loss of vitamin activity during processing.

The lower limit for the vitamin contents for this product is set to 90% of declared amounts. It can be seen that all the vitamins are well inside this limit which shows that the chosen overages for the vitamins are sufficient for a shelf life of 18 months at 20°C and ambient relative humidity.

All the other physical, microbiological and sensoric requirements were according to the required specification when analyzed after 18 months.

### EXAMPLE 3

### Physical Stability

A stability study was carried out in order to investigate the physical stability of the liquid multivitamin composition. Two production batches referred to in Example 2 were compared with 2 newly produced batches. The four products were all evaluated in the original packaging which is 500 ml polyethylene terephtalate and black coloured bottles.

The earlier produced batches had been stored at 25°C and 60% relative humidity.

The mean oil droplet size and the homogeneity of the emulsion was investigated by the aid of a Zeiss microscope (Axioskop) with a 40x magnification.

Pictures of the emulsions were taken with the aid of an Olympus DP 10 digital camera and the droplet sizes quantified with Olympus DP-software.

The following procedure was undertaken in order to investigate the homogeneity of the emulsion.

The liquid content of each bottle was poured out into 50 divided doses of 10 ml. The bottles were not shaken before the dispensing of the individual dosages of 10 ml which should represent the most challenging situation with respect to the detection of any emulsion inhomogeneity. The results are presented below.

### Results:

Samples were investigated under the microscope in order to evaluate the homogeneity at the top, in the middle and at the bottom of the contents in each bottle. Two separate measurements were carried out on each microscope slide preparation and the number and diameter of the oil droplets from 0.5µm and upwards notified.

The 10 ml samples from each bottle were investigated visually with respect to the presence of any oily film on the surface of the emulsion in each beaker. There was no evidence of an oil film in any of the examined beakers.

All the samples had a homogenous appearance.

The liquid emulsion from all the four batches was easily pourable in a smooth fashion without the presence of any lumps.

There was no evidence of any segregation with respect to the average oil droplet size or the droplet count in the samples taken at the top, middle and bottom of the product in any of the examined batches.

The mean oil droplet size for the four batches was in the range of 3.1 to 6.2 µm. There was no difference between the newly produced batches and the stability batches with respect to the mean oil droplet size.

There was no evidence of emulsion creaming or phase separation in any of the samples tested and there was no difference in this respect between newly produced batches and batches stored for stability studies at 25°C and 60% relative humidity.

This ensures that the product is homogenous with respect to the dosing of the lipophilic vitamins contained in the discontinuous phase of the product.

### EXAMPLE 4

### Preparation of 5000 ml of a liquid multivitamin and mineral syrup

### Composition:

| | |
|---|---|
| Vitamin oil mixture: | 17.5 g |
| DL-α-tocopherol acetate: | 9600 mg |
| Vitamin A palmitate: | 950 mg |
| Cholecalciferol concentrate: | 540 mg |
| Betacaroten 20% | 1500 mg |
| Betacaroten 30% | 3125 mg |
| Lecithin: | 752 mg |
| Vitamin powder mixture: | 34.84 g |
| Nicotinamide: | 19.2 g |
| Thiamine nitrate: | 1.60g |
| Riboflavine | 1.32g |
| Pyridoxine hydrochloride: | 1.06g |
| Folic acid: | 303 mg |
| Cyanocobalamin 0.1%: | 11.20g |
| Biotin | 109 mg |
| Dexpanthenol: | 9.54 g |
| Ascorbic acid: | 14 g |
| Sodium ascorbate: | 47.03 g |
| Ferrous sulphate: | 52.34 g |
| Zinc sulphate: | 36.88 g |
| Potassium iodide: | 123 mg |
| Di-sodium-pyrophosphate: | 74.06 g |
| Agar agar: | 4.13 g |
| Galactomannan mixture: | 17.34 g |
| Potassium sorbate: | 7.50 g |
| Apricot flavour: | 5.70 g |
| Fructose: | 55 g |
| Invert sugar: | 3350 g |
| Purified water: | 2274 g |

The manufacturing method is analogous to that in Example 1 except for the inclusion of the minerals. The di-sodium-pyrophosphate is dissolved in a batch of water and added to the main solution at a temperature of 40-45°C. Ferrous sulphate, zinc sulphate and potassium iodide are added to the same main solution when the temperature has fallen below 40°C.

The function of di-sodium-pyrophosphate is to act as a complexing agent with respect to the minerals. This minimises the metallic aftertase from the minerals and prevents any possible catalytic effect with respect to the degradation of the labile vitamins in solution.

The vitamin and mineral syrup has a fresh taste of apricot, a viscosity of 400-1200 mPa.s (cps) at 20°C, density of 1.19-1.23 g/cm³ and a pH of 3.0-3.5.

Two batches of emulsion were subjected to a stability trial as set out in Example 2 with the results set out in the table below.
The following results for the vitamin contents after 18 months were obtained for the two production batches respectively at 20°C/ambient RH:

| Vitamin | Declared | Overage | Nominal | Initial | Recovery | 18 months | Degraded |
|---|---|---|---|---|---|---|---|
| Retinol (IU/5ml) | 1500 | 30% | 1950 | 2145,2145 | 110.0% | 1869, 1862 | 13% |
| Betacaroten(IU/5ml) | 1500 | 30% | 1950 | 2172,2148 | 110.8% | 2315,2279 | +6.3% |
| Cholecalciferol (IU/5ml) | 400 | 35% | 540 | 502, 494 | 92.2% | 519, 502 | +2.5% |
| D-α-tocopherol (IU/5ml) | 10 | 20% | 12 | 12.5, 12.4 | 103.8% | 12.0, 11.9 | 4.0% |
| Nicotinamide (mg/5ml) | 16 | 20% | 19.2 | 19.5, 19.2 | 100.8% | 18.5, 18.4 | 4.7% |
| Thiamine (mg/5ml) | 1.0 | 30% | 1.3 | 1.27,1.25 | 96.9% | 1.16, 1.16 | 7.9% |
| Riboflavine (mg/5ml) | 1.2 | 10% | 1.32 | 1.25, 1.23 | 93.9%, | 1.19, 1.19 | 4.0% |
| Pantothenic acid (mg/5ml) | 6.0 | 70% | 10.2 | 10.4, 10.3 | 101.5% | 9.2, 9.2 | 11.1% |
| Ascorbic acid (mg/5ml) | 35 | 60% | 56 | 53.0, 52.0 | 93.8% | 35.7, 34.9 | 32.8% |
| Pyridoxine (mg/5ml) | 0.8 | 10% | 0.88 | 0.88,0.87 | 99.4% | 0.89,0.88 | +1.1% |
| Folic acid (µg/5ml) | 200 | 40% | 280 | 276,266 | 96.8% | 255, 244 | 7.9% |
| Biotin (µg/5ml) | 100 | 10% | 110 | 109, 116 | 102.3% | 103, 100 | 9.8% |
| Vitamm B₁₂ (µg/5ml) | 4 | 180% | 11.2 | 11.6, 11.8 | 104.5% | 9.7, 9.8 | 16.7% |
| Iodine (µg/5ml) | 90 | 5% | 94.5 | | | | |
| Iron (mg/5ml) | 10 | 5% | 10.5 | 10.9, 10.4 | 106.5% | 10.6, 10.6 | 0.5% |
| Zinc(mg/5ml) | 8 | 5% | 8.4 | 8.2, 8.2 | 102.5% | 8.4, 8.2 | 1.2% |

The averages in this example were set somewhat higher when compared to the formulation in Example 1. This was due to the fact that the lower limit for the vitamin contents for this product was set to 100% of declared amounts. It can be seen that all the vitamins are well inside this limit which shows that the chosen overages for the vitamins are sufficient for a shelf-life of 18 months at 20°C and ambient relative humidity.

Indeed the overages for all the vitamins can be reduced to the same levels as chosen in Example 1. Vitamin C is an exception and this overage has to be 60% in order to be 100% of the declared amount at the end of the shelf-life period when stored at 20°C and ambient relative humidity. An overage of 20% is sufficient for vitamin B₁₂ in order to ensure the declared amount at the end of the shelf life period (18 months).

All the other physical, microbiological and sensoric requirements were according to the specification when analysed after 18 months.

### EXAMPLES 5, 6, 7, 8 AND 9: Variation of Emulsifying Agent

The method of preparation as outlined in Example 1 was followed. The initial viscosity, mean oil droplet size and appearance were recorded.

The formulations were dispensed into 200 ml amber glass bottles and into clear glass test tubes which were capped. The bottles and test tubes were placed in the following conditions: 1) at 7°C and ambient humidity in a refrigerator; 2) 25°C and 60% RH; and 3) 30°C and 60% RH.

The test tubes were observed for any evidence of creaming or coalescence of the disperse phase after 30 days. The samples were also observed to see if any syneresis had taken place. Syneresis is a phenomenon that can occur in liquid formulations containing a gelling agent. It is thought to be due to changes taking place in the polymer network causing gel shrinkage which results in evolution of a clear aqueous phase devoid of the stabilising gelling and thickening agents. Samples exhibiting syneresis change to a normal homogenous appearance when samples are lightly shaken or bottles turned upside down.

The observations were documented by digital photographs of the test tubes in a cupboard with controlled light conditions.

Five trials were conducted with different types of emulsifying agents. These were Lutrol^{®} F68 or Poloxamer 188 from BASF^{®}, Polysorbate 80 from Dr. W. Kolb AG, Pemulen which is a polyacrylic acid polymer from BFGoodrich Speciality Chemicals, Tefose 1500 which is PEG-6 stearate from Gattefosse and Plurol stearique WL 1009 which is polyglyceryl-6 distearate from Gattefosse.

The emulsifying agent was divided between the oil phase and the aqueous phase in order to ease the dispersion and emulsification process. 100 mg of the emulsifying agent was dispersed in the aqueous phase in the case of Examples 5, 6, 8 and 9 and 50 mg was added to the aqueous phase in case of Example 7.

The composition and analytical results are given below.

| Ingredient | 5 | 6 | 7 | 8 | 9 |
|---|---|---|---|---|---|
| Vitamin oil mixture: | | | | | |
| DL-α-tocopherol acetate | 2.82 g | 2.82 g | 2.82 g | 2.82 g | 2.82 g |
| Vitamin A palmitate | 324 mg | 324 mg | 324 mg | 324 mg | 324 mg |
| Cholecalciferol concentrate | 99 mg | 99 mg | 99 mg | 99 mg | 99 mg |
| Medium chain triglycerides | 360 mg | 360 mg | 360 mg | 360 mg | 360 mg |
| Orange oil | 2.07 g | 2.07 g | 2.07 g | 2.07 g | 2.07 g |
| Betacaroten susp. 30% | 140 mg | 140 mg | 140 mg | 140 mg | 140 mg |
| Polysorbate 80 | 340 mg | | | | |
| Poloxamer 188 | | 340 mg | | | |
| Pemulen TR-2 | | | 98 mg | | |
| Plurol stearique | | | | 340 mg | |
| Tefose 1500 | | | | | 340 mg |
| Vitamin powder mixture: 6.0g | | | | | |
| Nicotinamide | 3.78 g | 3.78 g | 3.78 g | 3.78 g | 3.78 g |
| Pyridoxine hydrochloride | 459 mg | 459 mg | 459 mg | 459 mg | 459 mg |
| Thiamine nitrate | 367 mg | 367 mg | 367 mg | 367 mg | 367 mg |
| Riboflavine | 330 mg | 330 mg | 330 mg | 330 mg | 330 mg |
| Folic acid | 29 mg | 29 mg | 29 mg | 29 mg | 29 mg |
| Sorbitol powder | 1.04g | 1.04 g | 1.04 g | 1.04g | 1.04 g |
| Dexpanthenol | 1.236 g | 1.236 g | 1.236 g | 1.236 g | 1.236 g |
| Ascorbic acid | 16.89 g | 16.89 g | 16.89 g | 16.89 g | 16.89 g |
| Potassium sorbate | 2.1 g | 2.1 g | 2.1 g | 2.1 g | 2.1 g |
| Sorbitol 70% non crystalline | 2.13 kg | 2.13 kg | 2.13 kg | 2.13 kg | 2.13 kg |
| Agar agar | 2.44 g | 2.44 g | 2.44 g | 2.44 g | 2.44 g |
| Galactomannan mixture | 10.44 g | 10.44 g | 10.44 g | 10.44 g | 10.44 |
| Sorbitol powder | 33.6 g | 33.6 g | 33.6 g | 33.6 g | 33.6 g |
| Citric acid monohydrate | 12.18 g | 12.18 g | 12.18 g | 12.18 g | 12.18 g |
| Purified water to: | 3000 ml | 3000 ml | 3000 ml | 3000 ml | 3000 ml |
| | | | | | |
| Viscosity (mPa) | 892 | 1050 | 1050 | 964 | 968 |
| Mean oil droplet size (µn) | 2.7 | 3.2 | 4.7 | 3.7 | 1.9 |
| Mean oil droplet size (µm) 30 days | 3.8 | 3.2 | 4.1 | 3.0 | 3.7 |

From the results it can be seen, that all the batches contained a disperse phase with a satisfactory low value for the mean droplet size (in the range of 1-5 µm). Examples 5-9 all showed a satisfactory stability at all temperatures after 30 days. Their mean oil droplet diameter stayed unchanged and there was no evidence of any creaming or syneresis in any of the sample test tubes.

### EXAMPLES 10, 11 AND 12: Variation of Thickener

The method of preparation as outlined in Example 1 was followed. The initial viscosity, mean oil droplet size and appearance were recorded.

The formulations were dispensed into 200 ml amber glass bottles and into clear glass test tubes which were capped. The bottles and test tubes were placed in the following conditions: 1) at 7°C and ambient humidity in a refrigerator; 2) 25°C and 60% RH; and 3) 30°C and 60% RH.

The test tubes were observed for any evidence of creaming or coalescence of the disperse phase after 30 days. The samples were also observed to see if any syneresis had taken place. Syneresis is a phenomenon that can occur in liquid formulations containing a gelling agent. It is thought to be due to changes taking place in the polymer network causing gel shrinkage which results in evolution of a clear aqueous phase devoid of the stabilising gelling and thickening agents. Samples exhibiting syneresis change to a normal homogenous appearance when samples are lightly shaken or bottles turned upside down.

The observations were documented by digital photographs of the test tubes in a cupboard with controlled light conditions.

Three trials have been carried out to investigate different types of thickeners in combination with agar agar. The three thickeners used were Keltrol RD, which is a xanthan gum from Kelco UK Ltd., traganth from Agricales Ltd. and a combination of acacia gum and xanthan gum. 100 mg of the emulsifying agent was dispersed in the aqueous phase in all the examples.

The composition and analytical results are given below.

| Ingredients | 10 | 11 | 12 |
|---|---|---|---|
| Vitamin oil mixture: 6,05 g | | | |
| DL-α-tocopherol acetate | 2.82 g | 2.82 g | 2.82 g |
| Vitamin A palmitate | 324 mg | 324 mg | 324 mg |
| Cholecalciferol concentrate | 99 mg | 99 mg | 99 mg |
| Medium chain triglycerides | 360 mg | 360 mg | 360 mg |
| Orange oil | 2.07 g | 2.07 g | 2.07 g |
| Betacaroten susp. 30% | 140 mg | 140 mg | 140 mg |
| Emultop | 240 mg | 240 mg | 240 mg |
| Vitamin powder mixture: 6,0 g | | | |
| Nicotinamide | 3.78 g | 3.78 g | 3.78 g |
| Pyridoxine hydrochloride | 459 mg | 459 mg | 459 mg |
| Thiamine nitrate | 367 mg | 367 mg | 367 mg |
| Riboflavine | 330 mg | 330 mg | 330 mg |
| Folic acid | 29 mg | 29 mg | 29 mg |
| Sorbitol powder | 1.04 g | 1.04 g | 1.04 g |
| Dexpanthenol | 1.236 g | 1.236 g | 1.236 g |
| Ascorbic acid | 16.89 g | 16.89 g | 16.89 g |
| Potassium sorbate | 2.1 g | 2.1 g | 2.1 g |
| Sorbitol 70% non crystalline | 2.13 kg | 2.13 kg | 2.13 kg |
| Agar agar | 2.44 g | 2.44 g | 2.44 g |
| Keltrol RD | 10.44 g | | 9.00 g |
| Traganth gum | | 7.50 g | |
| Acacia gum | | | 3.00 g |
| Sorbitol powder | 33.6 g | 33.6 g | 33.6 g |
| Emultop | 100 mg | 100 mg | 100 mg |
| Citric acid monohydrate | 12.18 g | 12.18 g | 12.18 g |
| Purified water to: | 3000 ml | 3000 ml | 3000 ml |
| | | | |
| Viscosity (mPa) | 1110 mPa | 660 mPa | 1110 mPa |
| Mean oil droplet (µm) | 2.6 | 4.5 | 2.7 |
| Mean oil droplet (µm) 30 days | 2.7 | 3.7 | 2.6 |

Examples 10-12 all produced satisfactory results with respect to viscosity and mean oil droplet size of the disperse phase in the ranges of 600-1100 mPa and 2-5 µm respectively.

The stability results for Examples 10-12 after 30 days all showed excellent stability with respect to the mean droplet size and there was no evidence of creaming or syneresis at any temperature.

### EXAMPLES 13 AND 14

The method of preparation as outlined in Example 1 was followed. The initial viscosity, mean oil droplet size and appearance were recorded.

The formulations were dispensed into 200 ml amber glass bottles and into clear glass test tubes which were capped. The bottles and test tubes were placed in the following conditions: 1) at 7°C and ambient humidity in a refrigerator; 2) 25°C and 60% RH; and 3) 30°C and 60% RH.

The test tubes were observed for any evidence of creaming or coalescence of the disperse phase after 30 days. The samples were also observed to see if any syneresis had taken place. Syneresis is a phenomenon that can occur in liquid formulations containing a gelling agent. It is thought to be due to changes taking place in the polymer network causing gel shrinkage which results in evolution of a clear aqueous phase devoid of the stabilising gelling and thickening agents. Samples exhibiting syneresis change to a normal homogenous appearance when samples are lightly shaken or bottles turned upside down.

The observations were documented by digital photographs of the test tubes in a cupboard with controlled light conditions.

Two trials were carried out, each containing a lipophilic drug and an oil phase comprising 5 or 20 %. The drug chosen for exemplification was carbamazepine.

The composition and analytical results are given below.

| Ingredients | 13 | 14 |
|---|---|---|
| Carbamazepine | 375 mg | 1.5 g |
| Medium chain triglycerides | 144 g | 576 g |
| Betacaroten susp. 30% | 1 g | 1.5 g |
| Polysorbate 80 | 6 g | 24 g |
| Agar agar | 1.83 g | 1.83 g |
| Potassium sorbate | 2.1 g | 2.1 g |
| Polysorbate 80 | 500 mg | 500 mg |
| Galactomannan mixture | 7.83 g | 5.83 g |
| Sorbitol 70% non crystalline | 2.13 kg | 2.13 kg |
| Sorbitol powder | 33.6 g | 33.6 g |
| Emultop | 100 mg | 100 mg |
| Citric acid monohydride | 12.18 g | 12.18 g |
| Purified water to: | 3000 ml | 3000 ml |
| | | |
| Viscosity (mPa) | 632 mPa | 1890 mPa |
| Mean oil droplet size (µm) | 5.4 | 4.4 |
| Mean oil droplet size (µm) 30 days | 4.4 | 4.9 |

Examples 13 and 14 both showed satisfactory stability with respect to the mean droplet size. The formulation containing 5% oil phase had a homogenous appearance with no evidence of any creaming or syneresis at the three temperatures. Some syneresis had occurred for the formulation containing 20% oil phase in all three samples. The observed syneresis disappeared when the bottles were shaken lightly.

### EXAMPLE 15

### Preparation of a liquid emulsion contaiing a lipophilic drug substances

Composition:

| Ingredients | |
|---|---|
| Probucol | 6.00 g |
| Long chain triglycerides (Soyabean oil) | 137 g |
| Betacaroten susp. 30% | 1 g |
| Polysorbate 80 | 6 g |
| Agar agar | 1.83 g |
| Potassium sorbate | 2.1 g |
| Polysorbate 80 | 500 mg |
| Galactomannan mixture | 7.83 g |
| Sorbitol 70% non crystalline | 2.13 kg |
| Sorbitol powder | 33.6 g |
| Emultop | 100 mg |
| Citric acid monohydrate | 12.18 g |
| Purified water to: | 3000 ml |

The composition is prepared in an analogous manner to Example 1 with the probucol being dissolved in the oil phase by ultrasonification.

### EXAMPLE 16

### Preparation of a liquid emulsion containing a lipophilic drug substance

Composition:

| Ingredients | |
|---|---|
| Diazepam | 3.00 g |
| Medium chain triglycerides | 140 g |
| Betacaroten susp. 30% | 1 g |
| Polysorbate 80 | 6 g |
| Agar agar | 1.83 g |
| Potassium sorbate | 2.1 g |
| Polysorbate 80 | 500 mg |
| Galactomannan mixture | 7.83 g |
| Sorbitol 70% non crystalline | 2.13 kg |
| Sorbitol powder | 33.6 g |
| Emultop | 100 mg |
| Citric acid monohydrate | 12.18 g |
| Purified water to: | 3000 ml |

The composition is prepared in an analogous manner to Example 1 with the diazepam being dissolved in the oil phase by ultrasonification.

### EXAMPLE 17

### Preparation of a liquid emulsion contaiing a lipophilic drug substances

Composition:

| Ingredients | |
|---|---|
| Danazol | 600 mg |
| Long chain triglycerides (Soyabean oil) | 143 g |
| Betacaroten susp. 30% | 1 g |
| Polysorbate 80 | 6 g |
| Agar agar | 1.83 g |
| Potassium sorbate | 2.1 g |
| Polysorbate 80 | 500 mg |
| Galactomannan mixture | 7.83 g |
| Sorbitol 70% non crystalline | 2.13 kg |
| Sorbitol powder | 33.6 g |
| Emultop | 100 mg |
| Citric acid monohydrate | 12.18 g |
| Purified water to: | 3000 ml |

The composition is prepared in an analogous manner to Example 1 with the danazol being dissolved in the oil phase by ultrasonification.

### EXAMPLE 18

### Preparation of a liquid emulsion contaiing a lipophilic drug substances

Composition:

| Ingredients | |
|---|---|
| Halofantrine base | 15.0 g |
| Long chain triglycerides (Peanut oil) | 128 g |
| Betacaroten susp. 30% | 1 g |
| Polysorbate 80 | 6 g |
| Agar agar | 1. 83 g |
| Potassium sorbate | 2.1 g |
| Polysorbate 80 | 500 mg |
| Galactomannan mixture | 7.83 g |
| Sorbitol 70% non crystalline | 2.13 kg |
| Sorbitol powder | 33.6 g |
| Emultop | 100 mg |
| Citric acid monohydrate | 12.18 g |
| Purified water to: | 3000 ml |

The composition is prepared in an analogous manner to Example 1 with the halofantrine being dissolved in the oil phase by ultrasonification.

## Claims

1. A process for the preparation of a liquid emulsion composition having a continuous aqueous phase containing a gelling agent and a thickener and optionally a physiologically tolerable amount of at least one water soluble vitamin and a discontinuous oil phase, comprising at least one lipophilic vitamin and optionally an edible triglyceride, said emulsion composition further containing at least one emulsifying agent, said process comprising:
forming an aqueous composition comprising an aqueous solution of a gelling agent and a thickener and optionally at least one water soluble vitamin;
forming a water-immiscible liquid composition comprising at least one emulsifying agent and at least one lipophilic vitamin;
mixing said water-immiscible composition with at least part of said aqueous composition whereby to form an oil-in-water emulsion; and
if required mixing further components with said emulsion whereby to form said liquid emulsion composition;
and wherein the gelling agent is agar agar.

2. A process as claimed in claim 1, wherein said lipophilic vitamin is selected from the group consisting of vitamin E, vitamin A, vitamin K and/or vitamin D or combinations thereof.

3. A process as claimed in claim 1 or 2, wherein the aqueous phase further comprises at least one water soluble vitamin in the aqueous phase.

4. A process as claimed in claim 3, wherein said water soluble vitamin is selected from the group consisting of thiamine, riboflavin, niacin, nicotinamide, vitamin B₆ group, biotin, pantothenic acid, folic acid, pyridoxine, pyridoxal, pyridoxamine, inositol, vitamin B₁₂, choline and/or ascorbic acid or combinations thereof.

5. A process as defined in any preceding claim, wherein the liquid emulsion composition further comprises minerals.

6. A process as claimed in claim 5, wherein said minerals are selected from the group consisting of zinc, iron, calcium, iodine, magnesium and/or phosphorus.

7. A process as claimed in any preceding claim, wherein said thickener is an edible gum or a mixture of edible gums.

8. A process as claimed in claim 7, wherein said edible gum mixture is locust bean gum and guar gum.

9. A process as claimed in any preceding claim, wherein an edible triglyceride is present in the discontinuous oil phase.

10. A process as claimed in claim 9, wherein said edible triglyceride is fish oil or plant oil.

11. A process as claimed in any preceding claim, wherein the discontinuous oil phase comprises vitamin E.

12. A process as claimed in claim 11, wherein said vitamin E is in the form of α-tocopherol, α-tocopherol acetate, α-tocopherol acid succinate, vitamin E TPGS and/or tocotrienol.

13. A process as claimed in any preceding claim, wherein said emulsifying agent is phospholipid or fatty acid ester.

14. A process as claimed in claim 13, wherein said emulsifier is lecithin.

15. A process as claimed in any preceding claim, wherein a physiologically tolerable inorganic compound of a nanometer size is added to said oil phase.

16. A process as claimed in claim 15, wherein said inorganic compound is calcium carbonate.

17. A process according to any preceding claim, wherein said emulsion further comprises a non-vitamin drug substance.

## Patentansprüche

1. Verfahren zur Herstellung einer flüssigen Emulsionszusammensetzung mit einer kontinuierlichen wässrigen Phase, die ein Geliermittel und ein Verdickungsmittel und optional eine physiologisch tolerierbare Menge von mindestens einem wasserlöslichen Vitamin enthält, und einer diskontinuierlichen Ölphase, die mindestens ein lipophiles Vitamin und optional ein essbares Triglycerid umfasst, wobei die Emulsionszusammensetzung weiterhin mindestens einen Emulgator enthält, und wobei das Verfahren folgendes umfasst:
Bilden einer wässrigen Zusammensetzung, die eine wässrige Lösung eines Geliermittels und eines Verdickungsmittels und optional mindestens ein wasserlösliches Vitamin umfasst;
Bilden einer mit Wasser nicht mischbaren Flüssigkeitszusammensetzung, die mindestens einen Emulgator und mindestens ein lipophiles Vitamin umfasst;
Mischen der mit Wasser nicht mischbaren Zusammensetzung mit mindestens einem Teil der wässrigen Zusammensetzung, um eine Öl-in-Wasser-Emulsion zu bilden; und
falls erforderlich Mischen von weiteren Komponenten mit der Emulsion, um die flüssige Emulsionszusammensetzung zu bilden;
und worin das Geliermittel Agar Agar ist.

2. Verfahren nach Anspruch 1, worin das lipophile Vitamin aus der Gruppe bestehend aus Vitamin E, Vitamin A, Vitamin K und/oder Vitamin D oder Kombinationen davon ausgewählt ist.

3. Verfahren nach Anspruch 1 oder 2, worin die wässrige Phase weiterhin mindestens ein wasserlösliches Vitamin in der wässrigen Phase umfasst.

4. Verfahren nach Anspruch 3, worin das wasserlösliche Vitamin aus der Gruppe bestehend aus Thiamin, Riboflavin, Niacin, Nicotinamid, Vitamin B₆-Gruppe, Biotin, Panthotensäure, Folsäure, Pyridoxin, Pyridoxal, Pyridoxamin, Inositol, Vitamin B₁₂, Cholin und/oder Ascorbinsäure oder Kombinationen davon ausgewählt ist.

5. Verfahren wie in irgendeinem der vorhergehenden Ansprüche definiert, worin die flüssige Emulsionszusammensetzung weiterhin Mineralien umfasst.

6. Verfahren nach Anspruch 5, worin die Mineralien aus der Gruppe bestehend aus Zink, Eisen, Kalzium, Iod, Magnesium und/oder Phosphor ausgewählt ist.

7. Verfahren nach irgendeinem der vorhergehenden Ansprüche, worin das Verdickungsmittel ein essbares Gummi oder eine Mischung aus essbaren Gummis ist.

8. Verfahren nach Anspruch 7, worin die essbare Gummimischung Johannisbrotgummi und Guargummi ist.

9. Verfahren nach irgendeinem der vorhergehenden Ansprüche, worin ein essbares Triglycerid in der diskontinuierlichen Ölphase vorkommt.

10. Verfahren nach Anspruch 9, worin das essbare Triglycerid Fischöl oder Pflanzenöl ist.

11. Verfahren nach irgendeinem der vorhergehenden Ansprüche, worin die diskontinuierliche Ölphase Vitamin E umfasst.

12. Verfahren nach Anspruch 11, worin das Vitamin E in der Form von α-Tocopherol, α-Tocopherolacetat, α-Tocopherolhydrogensuccinat, Vitamin E TPGS und/oder Tocotrienol ist.

13. Verfahren nach irgendeinem der vorhergehenden Ansprüche, worin der Emulgator Phospholipid oder Fettsäureester ist.

14. Verfahren nach Anspruch 13, worin der Emulgator Lecithin ist.

15. Verfahren nach irgendeinem der vorhergehenden Ansprüche, worin eine physiologisch tolerierbare unorganische Verbindung von einer Nanometergröße der Ölphase zugeführt wird.

16. Verfahren nach Anspruch 15, worin die unorganische Verbindung Kalziumcarbonat ist.

17. Verfahren nach irgendeinem der vorhergehenden Ansprüche, worin die Emulsion weiterhin eine nicht Vitamin Arzneimittelsubstanz umfasst.

## Revendications

1. Procédé pour la préparation d'une composition d'émulsion liquide présentant une phase aqueuse continue, contenant un gélifiant, un épaississant et, à titre facultatif, une quantité physiologiquement tolérable d'au moins une vitamine hydrosoluble, ainsi qu'une phase huileuse discontinue, comprenant au moins une vitamine lipophile et, à titre facultatif, un triglycéride comestible, ladite composition d'émulsion contenant en outre au moins un émulsifiant, ledit procédé comprenant :
la formation d'une composition aqueuse comprenant une solution aqueuse d'un gélifiant, d'un épaississant et, à titre facultatif, d'au moins une vitamine hydrosoluble ;
la formation d'une composition liquide non miscible à l'eau, comprenant au moins un émulsifiant et au moins une vitamine lipophile ;
le mélange de ladite composition non miscible à l'eau avec au moins une partie de ladite composition aqueuse, de façon à former une émulsion d'huile dans l'eau ; et
au besoin, le mélange d'autres composants avec ladite émulsion, de façon à former ladite composition d'émulsion liquide ;
et dans lequel le gélifiant est un agar-agar.

2. Procédé selon la revendication 1, dans lequel ladite vitamine lipophile est sélectionnée dans le groupe constitué de la vitamine E, de la vitamine A, de la vitamine K et/ou de la vitamine D ou des combinaisons de celles-ci.

3. Procédé selon la revendication 1 ou 2, dans lequel la phase aqueuse comprend en outre au moins une vitamine hydrosoluble dans la phase aqueuse.

4. Procédé selon la revendication 3, dans lequel ladite vitamine hydrosoluble est sélectionnée dans le groupe constitué de la thiamine, de la riboflavine, de la niacine, du nicotinamide, du groupe de la vitamine B₆, de la biotine, de l'acide pantothénique, de l'acide folique, de la pyridoxine, du pyridoxal, de la pyridoxamine, de l'inositol, de la vitamine B₁₂, de la choline et/ou de l'acide ascorbique ou des combinaisons de ceux-ci.

5. Procédé selon l'une des revendications précédentes, dans lequel la composition d'émulsion liquide comprend en outre des minéraux.

6. Procédé selon la revendication 5, dans lequel lesdits minéraux sont sélectionnés dans le groupe constitué du zinc, du fer, du calcium, de l'iode, du magnésium et/ou du phosphore.

7. Procédé selon l'une des revendications précédentes, dans lequel ledit épaississant est une gomme comestible ou un mélange de gommes comestibles.

8. Procédé selon la revendication 7, dans lequel ledit mélange de gommes comestibles est une gomme de caroube et une gomme de guar.

9. Procédé selon l'une des revendications précédentes, dans lequel un triglycéride comestible est présent dans la phase huileuse discontinue.

10. Procédé selon la revendication 9, dans lequel ledit triglycéride comestible est une huile de poisson ou une huile végétale.

11. Procédé selon l'une des revendications précédentes, dans lequel la phase huileuse discontinue comprend la vitamine E.

12. Procédé selon la revendication 11, dans lequel ladite vitamine E se présente sous la forme d'α-tocophérol, d'acétate d'α-tocophérol, de succinate acide d'α-tocophérol, de vitamine E TPGS et/ou de tocotriénol.

13. Procédé selon l'une des revendications précédentes, dans lequel ledit émulsifiant est un phospholipide ou un ester d'acides gras.

14. Procédé selon la revendication 13, dans lequel ledit émulsifiant est la lécithine.

15. Procédé selon l'une des revendications précédentes, dans lequel un composé inorganique physiologiquement tolérable de taille nanométrique est ajouté à ladite phase huileuse.

16. Procédé selon la revendication 15, dans lequel ledit composé inorganique est le carbonate de calcium.

17. Procédé selon l'une des revendications précédentes, dans lequel ladite émulsion comprend en outre une substance médicamenteuse non vitaminique.
